# EUROPEAN PATENT APPLICATION

(11) **EP 4 039 671 A1**
(43) Date of publication of application: **10.08.2022**
(21) Application number: 21155686.5
(22) Date of filing: 08.02.2021
(51) Int. Cl.: C07C 45/45, C07C 45/67

(54) **MANUFACTURING OF UNSATURATED KETONES USING AN ADDUCT OF A TERTIARY AMINE WITH SO3**

(71) Applicant: DSM IP Assets B.V., 6411 TE Heerlen (NL)
(72) Inventor: AQUINO, Fabrice, 4303 Kaiseraugst (CH); BONRATH, Werner, 4303 Kaiseraugst (CH)
(74) Representative: Dux, Roland

(57) **Abstract**

The present invention relates to a process of manufacturing of an unsaturated ketone of the formula (I) using adducts of tertiary amines and SO₃ The reaction provides the unsaturated ketones in high yields and selectivities.

## Description

### Technical Field

The present invention relates to the manufacturing of unsaturated ketones.

### Background of the invention

Unsaturated ketones of the formula (I) are an important class of industrial chemicals and are central products in the synthesis of vitamins and aroma ingredients. particularly important. One of the possible synthetic routes uses tertiary propargyl carbinols respectively tertiary vinyl carbinols as starting products.

US 3,029,287 and G. Saucy et al. disclose in Helv. Chim. Acta 1967, 50(4), 1158-1167 discloses the condensation of a tertiary propargyl alcohol and a ketal or an enol ether to form an allenic ketone in the presence of strong acids, particularly sulfuric acid or phosphoric acid or p-toluenesulfonic acid.

US 6,380,437 discloses the condensation of a tertiary propargyl alcohol and a ketal or an alkenyl alkyl ether to form an allenic ketone in the presence of an aliphatic sulfonic acid or a metal salt thereof.

US 3,330,867 and WO 2017/131607 disclose that an allene ketone can be isomerized to a unsaturated ketone with hydrogen.

The use of strong acid in the preparation of allene ketones is disadvantageous as these chemicals are hazardous in the handling and use special protection methods and require specific and corrosion-resistant materials for the equipment used in the manufacturing process.

G. Saucy et al. disclose in Helv. Chim. Acta 1967, 50, 2091-2095 and Helv. Chim. Acta 1967, 50, 2095-2100 that phosphoric acid or sulfuric acid or p-toluene sulfonic acid can be used as catalyst for the reaction between tertiary vinyl carbinols and isopropenyl ethers.

DE 196 49 564 discloses that for this reaction diverse organophosphorous compounds can be used as catalysts.

WO 2010/046199 A2 discloses inorganic ammonium (NH₄⁺) salts as potential catalysts for this reaction.

However, inorganic ammonium salts have typically a disadvantageous solubility and it been shown that the inorganic ammonium salts show lower yields and selectivities particularly when used at concentrations of less than 1 mol-%.

### Summary of the invention

Therefore, the problem to be solved by the present invention is to offer a process of manufacturing of unsaturated ketones of the formula (I) in high yield under the absence of strong acids and corrosive conditions and.

Surprisingly, it has been found that a process according to the claim 1 and the reaction mixture according to claims 13 and 14 are able to solve this problem.

It has been found that adducts of tertiary amines and SO₃ are particularly well suited as catalyst for said reaction.

Further aspects of the invention are subject of further independent claims. Particularly preferred embodiments are subject of dependent claims.

### Detailed description of the invention

In a first aspect, the present invention relates to a process for the manufacture of an unsaturated ketone of the formula (I) 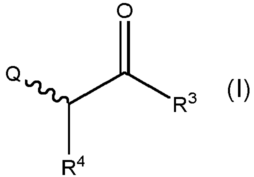 by reaction of
a compound of the formula (IIa) or (IIb) with a compound of the formula (IIIa) or (IIIb) 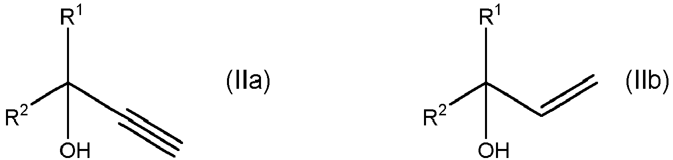 with a compound of the formula (IIIa) or (IIIb) 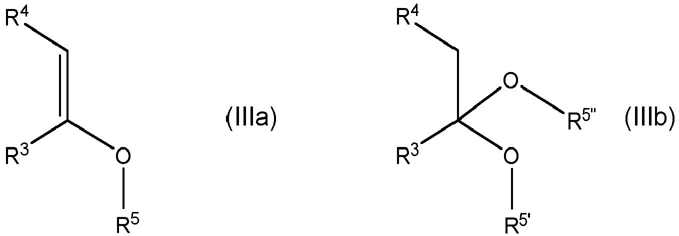 in the presence of an adduct of a tertiary amine with SO₃ as catalyst; wherein
Q represents a group of the formula 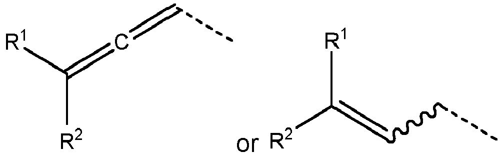
R¹ represents a methyl or ethyl group;
R² represents a saturated or unsaturated linear or branched or cyclic hydrocarbyl group with 1 to 46 C atoms;
R³ represents a methyl or an ethyl group;
R⁴ represents H or methyl or an ethyl group;
R⁵ represents a linear or branched C₁₋₁₀-alkyl group, particularly a methyl or an ethyl group;
R^{5'} and R^{5"} represent
   either a linear or branched C₁₋₁₀-alkyl group, particularly a methyl or an ethyl group;
   or R^{5'} and R^{5"} form together a linear or branched C₁₋₁₀-alkylene group, particularly an ethylene or propylene group;
   and
wherein the tertiary amine of said adduct is
   either
   pyridine
   or
   a tertiary amine of the formula (X) 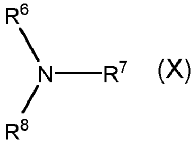 wherein R⁶ and R⁷ and R⁸ represent independently from each a linear or branched C₁₋₁₀-alkyl group
   and
wherein the wavy line represents a carbon-carbon bond and which when linked to the carbon-carbon double bond is either in the Z or in the E-configuration;
and any dotted line represents the bond by which the substituent Q is bound to the rest of the compound of the formula (I).

For sake of clarity, some terms used in the present document are defined as follows:
In the present document, a "C_{x-y}-alkyl" group is an alkyl group comprising x to y carbon atoms, i.e., for example, a C₁₋₃-alkyl group is an alkyl group comprising 1 to 3 carbon atoms. The alkyl group can be linear or branched. For example -CH(CH₃)-CH₂-CH₃ is considered as a C₄-alkyl group.

In case identical labels for symbols or groups are present in several formulae, in the present document, the definition of said group or symbol made in the context of one specific formula applies also to other formulae which comprises the same said label.

The term "independently from each other" in this document means, in the context of substituents, moieties, or groups, that identically designated substituents, moieties, or groups can occur simultaneously with a different meaning in the same molecule.

In the present document, any dotted line in formulae represents the bond by which a substituent is bound to the rest of a molecule.

The term "tertiary amine" relates in this document to a compound having at least one nitrogen atom which is bound to 3 carbon atoms. Said bonds can be to 3 different carbon atoms by aliphatic bonds or they may be olefinic or aromatic. In case of aromatic or olefinic bonds, two of the 3 bonds are to the same carbon atom. Hence, triethyl amine, 1,8-diazabicyclo[5.4.0]undec-7-ene (=DBU) and pyridine are, hence , all example for tertiary amines in the sense of the present invention.

Any wavy line in any formula of in this document represents a carbon-carbon bond and which when linked to the carbon-carbon double bond is either in the Z or in the E-configuration. It is preferred in all molecules that the carbon-carbon double bond is in the E-configuration.

The unsaturated ketone of the formula (I) encompasses two variants, i.e. the compound of the formula (I') and the compound of the formula (I") 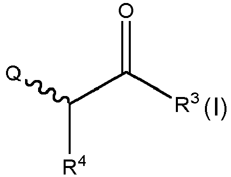 with Q being

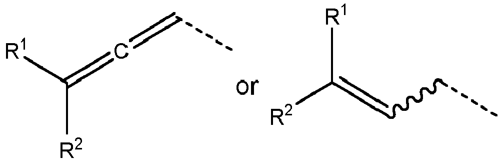

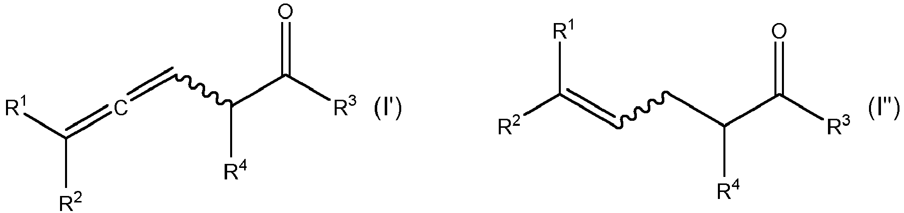

### Compound of the formula (I')

The compound of the formula (I') is prepared by reaction of the compound of the formula (IIa) and a compound of the formula (IIIa) or of the formula (IIIb), preferably of the formula (IIIa), in the presence of an adduct of a tertiary amine with SO₃ as catalyst (="***Cat***") as discussed later-on in great detail:

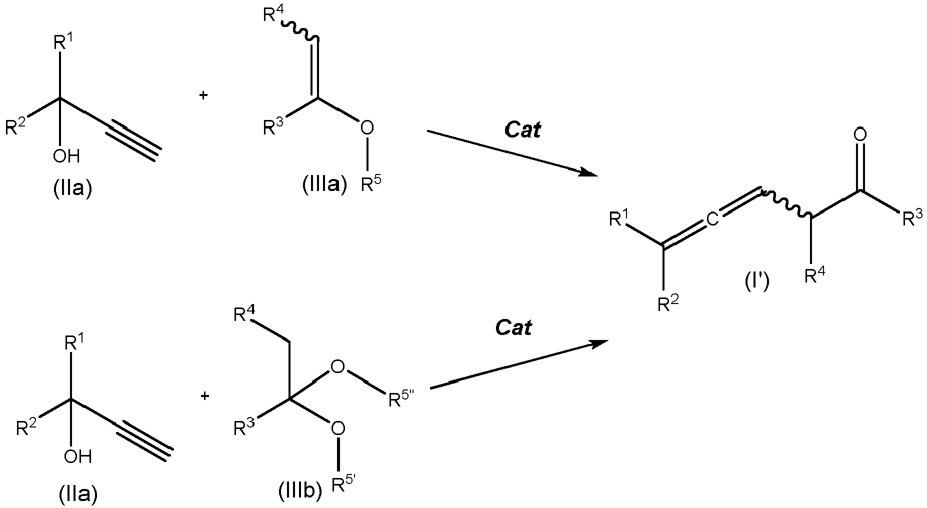

According to this embodiment, the compound of the general formula (I) is preferably selected from the group consisting of 6-methylhepta-4,5-dien-2-one, 6,10-dimethylundeca-4,5-dien-2-one, 6,10-dimethylundeca-4,5,9-trien-2-one, 6,10,14-trimethylpentadeca-4,5,9,13-tetraen-2-one, 6,10,14-trimethylpentadeca-4,5,9-trien-2-one, 6,10,14-trimethylpentadeca-4,5,13-trien-2-one and 6,10,14-trimethylpentadeca-4,5-dien-2-one.

### Compound of the formula (I")

The compound of the formula (I") is prepared by reaction of a compound of the formula (IIb) and a compound of the formula (IIIa) or of the formula (IIIb), preferably of the formula (IIIa), in the presence of an adduct of a tertiary amine with SO₃ as catalyst (="***Cat***") as discussed later-on in great detail:

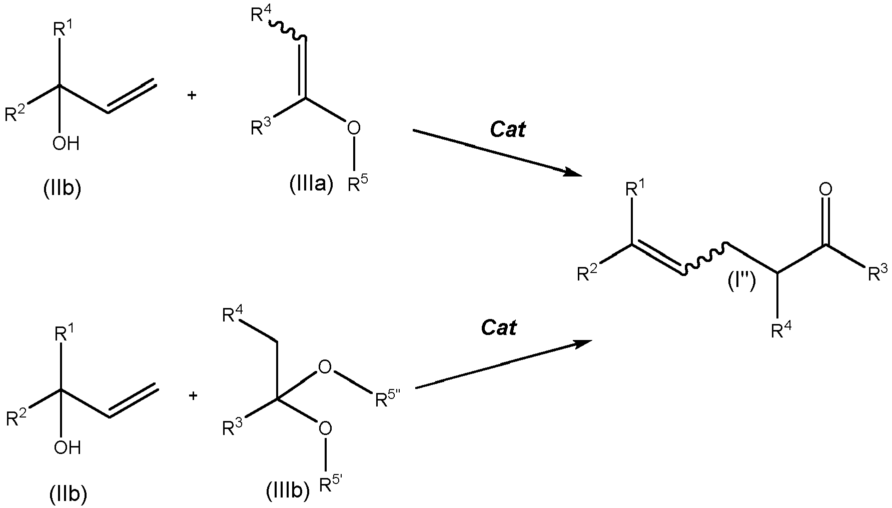

According to this embodiment, the compound of the general formula (I) is preferably selected from the group consisting of 6-methyl-5-hepten-2-one, 6-methyl-5-octen-2-one, 6,10-dimethyl-5,9-undecadien-2-one, 6,10-dimethyl-5-undecen-2-one, 6,10,14-trimethylpentadeca-5,9-dien-2-one, 6,10,14-trimethylpentadeca-5,13-dien-2-one and 6,10,14-trimethyl-5,9,13-pentadeca-trien-2-one, preferably 6-methyl-5-hepten-2-one.

### Compound of the formula (IIa) and (IIb)

The compounds of the formula (IIa) and (IIb) are substances known to the person skilled in the art.

R¹ represents a methyl or ethyl group, preferably a methyl group.

R² represents a saturated or unsaturated linear or branched or cyclic hydrocarbyl group with 1 to 46 C atoms, preferably a methyl group.

In a preferred embodiment, R² represents a group which is selected from the group consisting of the formula (R2-I), (R2-II), (R2-III) and (R2-IV)

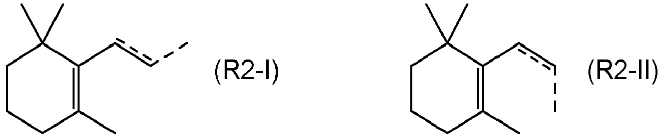

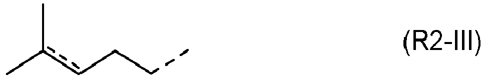

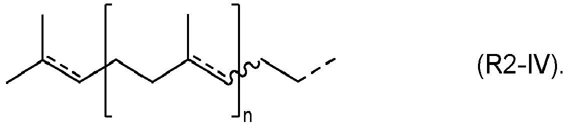

The dotted line represents the bond by which the substituent of the formula (R2-I), (R2-II), (R2-III) or (R2-IV) is bound to the rest of the compound of the formula (I) or formula (IIa) or (IIb). Any double bond having dotted line (------) represents independently from each other either a single carbon-carbon bond or a double carbon-carbon bond. Any wavy line represents independently from each other a carbon-carbon bond which when linked to the carbon-carbon double bond is either in the Z or in the E-configuration.

In the above formulae n represents 1, 2, 3 or 4, particularly 1 or 2.

In one of the preferred embodiments, R² represents either a group of the formula (R2-I) or of the formula (R2-II).

In another preferred embodiment, R² represents either a group of the formula (R2-III) or of the formula (R2-IV).

It is preferred that compound of the formula (IIa) is selected from the group consisting of 2-methylbut-3-yn-2-ol (=methylbutynol, *"MBY"*), 3-methylpent-1-yn-3-ol (=ethylbutynol, *"EBY"*), 3,5-dimethylhex-1-yn-3-ol, 3,7-dimethyloct-6-en-1-yn-3-ol (= dehydrolinalool, *"DLL"*), 3,7-dimethyloct-1-yn-3-ol, 3,7-dimethylnon-6-en-1-yn-3-ol (= ethyldehydrolinalool, *"EDLL"*), 3,7,11-trimethyldodec-1-yn-3-ol, 3,7,11-trimethyldodec-6-en-1-yn-3-ol, 3,7,11-trimethyldodeca-6,10-dien-1-yn-3-ol (= dehydronerolidol, *"DNL"*), 3,7,11-trimethyldodeca-4,6,10-trien-1-yn-3-ol (= ethinylpseudoionol, *"EPI*")*,* 3-methyl-5-(2, 6, 6-trimethylcyclohex-1-en-1-yl)pent-1-yn-3-ol and 3-methyl-1-(2,6,6-trimethylcyclohex-1-en-1-yl)pent-1-en-4-yn-3-ol.

It is particularly preferred that compound of the formula (IIa) is selected from the group consisting of 2-methylbut-3-yn-2-ol (=methylbutynol, *"MBY*"), *3-*methylpent-1-yn-3-ol (=ethylbutynol, *"EBY"*), 3,7-dimethyloct-6-en-1-yn-3-ol (= dehydrolinalool, *"DLL"*), 3,7-dimethylnon-6-en-1-yn-3-ol (= ethyldehydrolinalool, *"EDLL"*) ,3,7,11-trimethyldodeca-6,10-dien-1-yn-3-ol (= dehydronerolidol, *"DNL"*) and 3,7,11-trimethyldodec-1-yn-3-ol.

It is even more preferred that the compound of the formula (IIa) is selected from the group consisting of 3,7-dimethyloct-6-en-1-yn-3-ol (= dehydrolinalool, *"DLL"*), 3,7-dimethylnon-6-en-1-yn-3-ol (= ethyldehydrolinalool, *"EDLL"*) and 3,7,11-trimethyldodec-1-yn-3-ol.

It is particular preferred that compound of the formula (IIb) is selected from the group consisting of 2-methyl-3-buten-2-ol ("*MBE"*), 3-methyl-4-penten-3-ol (*"EBE"*), 3,7-dimethyl-1,6-octadien-3-ol (= linalool, *"LL"*), 3,7-dimethyl-1-octen-3-ol (*"DMOE"*) and 3,7,11-trimethyl-1,6-dodecadien-3-ol (nerolidol, "*NL*"), particularly (6E)-3,7,11-trimethyl-1,6-dodecadien-3-ol (= E-nerolidol, *"E-NL"*)*.*

It is even more preferred that compound of the formula (IIb) is selected from the group consisting of 2-methyl-3-buten-2-ol ("*MBE*"), 3,7-dimethyl-1,6-octadien-3-ol (= linalool, "*LL*"), 3,7-dimethyl-1-octen-3-ol ("*DMOE*") and 3,7,11-trimethyl-1,6-dodecadien-3-ol (nerolidol, "*NL*"), particularly (6E)-3,7,11-trimethyl-1,6-dodecadien-3-ol (= E-nerolidol, *"E-NL"*).

It is even more preferred that the compound of the formula (IIb) is 2-methyl-3-buten-2-ol (*"MBE"*) or 3,7-dimethyl-1,6-octadien-3-ol (= linalool, *"LL"*)*.*

### Compound of the formula (IIIa)

The compounds of the formula (IIIa) are substances known to the person skilled in the art.

In formula (IIIa) R³ represents a methyl or an ethyl group and R⁴ represents H or methyl or an ethyl group and R⁵ represents R⁵ represents a linear or branched C₁₋₁₀-alkyl group, particularly a methyl or an ethyl group.

Preferably, the group R³ represents a methyl group.

Preferably, the group R⁴ represents H.

Preferably, the group R⁵ represents a methyl group.

The compound of the formula (IIIa) is most preferably either isopropenyl methyl ether (*"IPM"*) or isopropenyl ethyl ether ("*IPE*"), particularly isopropenyl methyl ether (*"IPM"*).

Due to the synthesis of compound of the formula (IIIa), very often also mixtures of compounds of the formula (IIIa) are used for the reaction with compound of the formula (IIa) or (IIb). For example, for butenyl methyl ether, often a mixture of 2-methoxybut-1-ene and (E)-2-methoxybut-2-ene and (Z)-2-methoxy-but-2-ene being prepared from methanol and methyl ethyl ketone, is used.

### Compound of the formula (IIIb)

The compounds of the formula (IIIb) are substances known to the person skilled in the art.

In formula (IIIb) R³ represents a methyl or an ethyl group and R⁴ represents H or methyl or an ethyl group.

R^{5'} and R^{5"} represent in one embodiment each either a linear or branched C₁₋₁₀-alkyl group, particularly a methyl or an ethyl group. In another embodiment, R^{5'} and R^{5"} form together a linear or branched C₁₋₁₀-alkylene group, particularly an ethylene or propylene group.

Preferably, the group R³ represents a methyl group.

Preferably, the group R⁴ represents H.

In one preferred embodiment, R^{5'} = R^{5"} and particularly R^{5'} = R^{5"} = methyl or ethyl, more preferably R^{5'} = R^{5"} = CH₃.

In another preferred embodiment, R^{5'} and R^{5"} form together an ethylene (CH₂CH₂) or propylene (CH₂CH₂CH₂ or CH(CH₃)CH₂) group.

The compound of the formula (IIIb) is most preferably either 2,2-dimethoxypropane or 2,2-diethoxypropane or 2,2-dimethyl-1,3-dioxolane or 2,2,4-trimethyl-1,3-dioxolane or 2,2-dimethyl-1,3-dioxane.

The compound of the formula (IIIb) is most preferably either 2,2-dimethoxypropane or 2,2-diethoxypropane, particularly 2,2-dimethoxypropane.

The use of the compound of formula (IIIa) is preferred over the compound of the formula (IIIb).

### Adduct of a tertiary amine with SO₃

The reaction of a compound of the formula (IIa) or (IIb) with a compound of the formula (IIIa) or (IIIb) is performed in the presence of an adduct of a tertiary amine with SO₃ as catalyst.

Principally, any tertiary amine, which does not have apart from the tertiary amine any further SO₃ reactive group in its structure, is suitable as tertiary amine for the formation of an adduct with SO₃. Of course, tertiary amines, which have a molecular structure that sterically hinders the accessibility of the nitrogen electron lone pair to the SO₃ molecule are less suitable.

Adducts of tertiary amines are known to the person skilled in the art, and can be easily prepared from the tertiary amine and SO₃, as used in T.T. Tidwell, Synthesis (Germany), 1990, 1990(10):857-870.

Particularly suitable as tertiary amine for the SO₃-adduct are in one embodiment pyridinic compounds of the formula (V) 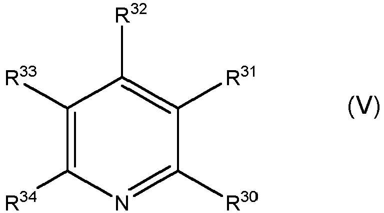 wherein R³⁰, R³¹, R³², R³³ and R³⁴ represent independently from each other either H or a linear or branched C₁₋₁₂-alkyl or C₅₋₁₂-cycloalkyl group.

It is preferred that R³⁰=R³¹=R³²=R³³, preferably R³⁰=R³¹=R³²=R³³=H.

It is further preferred that one of the groups of R³⁰, R³¹, R³², R³³ and R³⁴ is a methyl group.

It is also preferred that R³⁴ represents a methyl group or H, preferably H.

Most preferred as tertiary amines of the formula (V) are pyridine or α-picoline or β-picoline or γ-picoline. The most preferred is pyridine.

Hence, in one of the preferred embodiment the adduct of a tertiary amine with SO₃ is

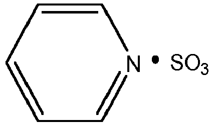

Particularly suitable as tertiary amine for the SO₃-adduct are in another embodiment trialkyl amines. Particularly suitable as trialkyl amines are amines having in alpha position to the nitrogen a CH₂ group, preferably triethyl amine.

Hence, in another preferred embodiment the adduct of a tertiary amine with SO₃ is

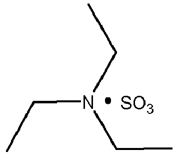

Further particularly suitable as tertiary amines are bridged tertiary amines, particularly the bridged tertiary amines selected from the group consisting of 1,8-diazabicyclo[5.4.0]undec-7-ene (=DBU), 1,5-diazabicyclo[4.3.0]non-5-ene (=DBN), 1,4-diazabicyclo[2.2.2]octane (=DABCO), 1-azabicyclo[2.2.2]octane (=quinuclidine) and sparteine, particularly 1,4-diazabicyclo[2.2.2]octane (=DABCO) or 1-azabicyclo[2.2.2]octane (=quinuclidine).

It has been found that the reaction is preferably performed when the molar ratio of the compound of the formula (IIa) or (IIb) to the compound of the formula (IIIa) or (IIIb) is ranging from 1:15 to 1:1.

In case of using the compound of the formula (IIIa), said ratio is more preferred in the range from 1:5 to 1:2, more preferably ranging from 1:3.5 to 1:2, most preferably ranging from 1:3 to 1:2, particularly ranging from 1:2.5 to 1:2.

In case of use of the compound of the formula (IIIb) said ratio is more preferred in the range from 1:10 to 1:2, more preferably ranging from 1:8 to 1:3, most preferably ranging from 1:8 to 1:5.

Furthermore, it is preferred that the amount the catalyst, i.e. the adduct of a tertiary amine with SO₃, is ranging from 0.005 - 1 mol-%, preferably ranging from 0.01 - 0.6 mol-%, more preferably ranging from 0.005 - 0.6 mol-%, most preferably ranging from 0.01 - 0.15 mol-%. based on the amount of the compound of the formula (IIa) respectively (IIb).

The reaction is preferably carried out at a temperature ranging from 70 to 170°C. In one embodiment, the temperature is preferably ranging from 110 to 160°C, most preferably at a temperature ranging from 115 to 150°C. This temperature range is particularly suitable for isopropenyl methyl ether as compound of the formula (IIIa).

In another embodiment, the temperature is preferably ranging from 75 to 100°C, most preferably at a temperature ranging from 80 to 95°C. This temperature range is particularly suitable for butenyl methyl ether as compound of the formula (IIIa).

The reaction is preferably carried out at a pressure ranging from 5 to 20 bar, more preferably at a pressure ranging from 6 to 15 bar.

The reaction is in one embodiment preferably carried out at a pressure ranging from 5 to 20 bara, more preferably at a pressure ranging from 6 to 15 bara. This pressure range is particularly suitable for isopropenyl methyl ether as compound of the formula (IIIa).

The reaction is in another embodiment preferably carried out at ambient pressure (1 bara). This pressure is particularly suitable for butenyl methyl ether as compound of the formula (IIIa).

The reaction can be carried out without solvent or in the presence of an organic solvent. Preferably the reaction is carried out without solvent.

Even if the reaction is carried out in the absence of an organic solvent, the starting materials, the compounds of the formula (IIa) or (IIb) or (IIIa) or (IIIb), may still be provided in an organic solvent. Thus, there may be an amount of organic solvent up to 10 weight-%, preferably an amount of organic solvent up to 5 weight-%, more preferably an amount of organic solvent up to 3 weight-%, based on the total weight of the reaction mixture.

If the reaction is carried out in an organic solvent, polar aprotic organic solvents such as aliphatic ketones as e.g. acetone are preferred.

It has been found that the above reaction provides the compound of the formula (I) in high conversion, yield and selectivity.

It has been found that particularly the compound of the formula (I) being selected from the group consisting of 6-methylhepta-4,5-dien-2-one, 6,10-dimethylundeca-4,5-dien-2-one, 6,10-dimethylundeca-4,5,9-trien-2-one, 6,10,14-trimethylpentadeca-4,5,9,13-tetraen-2-one, 6,10,14-trimethylpentadeca-4,5,9-trien-2-one, 6,10,14-trimethylpentadeca-4,5,13-trien-2-one and 6,10,14-trimethylpentadeca-4,5-dien-2-one can be preferably produced.

The reaction mixture itself, with or without an organic solvent, is also an object of the present invention.

Thus, the present invention relates in a further aspect to a reaction mixture comprising
i) a compound of the formula (IIa), 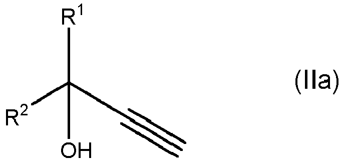
ii) a compound of the formula (IIIa) or (IIIb) 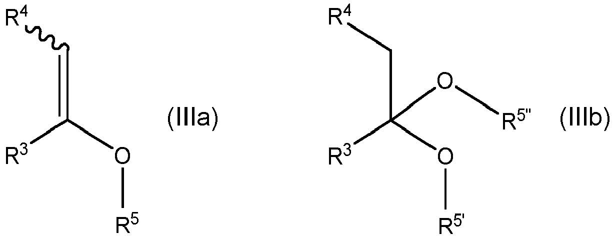 and
iii) an adduct of a tertiary amine with SO₃ as catalyst,
wherein the tertiary amine of the adduct is
either
a pyridinic compound of the formula (V) 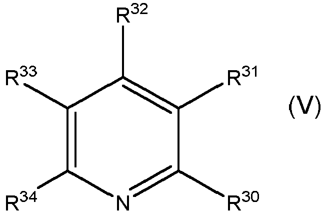 wherein R³⁰, R³¹, R³², R³³ and R³⁴ represent independently from each other either H or a linear or branched C₁₋₁₂-alkyl or C₅₋₁₂-cycloalkyl group;
or
a tertiary amine of the formula (X) 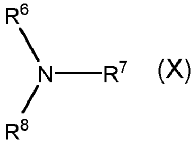 wherein R⁶ and R⁷ and R⁸ represent independently from each a linear or branched C₁₋₁₀-alkyl group
wherein
R¹ represents a methyl or ethyl group;
R² represents a saturated or unsaturated linear or branched or cyclic hydrocarbyl group with 1 to 46 C atoms;
R³ represents a methyl or an ethyl group;
R⁴ represents H or methyl or an ethyl group;
R⁵ represents a linear or branched C₁₋₁₀-alkyl group, particularly a methyl or an ethyl group;
R^{5'} and R^{5"} represent
   either a linear or branched C₁₋₁₀-alkyl group, particularly a methyl or an ethyl group;
   or R^{5'} and R^{5"} form together a linear or branched C₁₋₁₀-alkylene group, particularly an ethylene or propylene group.

The compound of the formula (IIa) and of the formula (IIIa) or (IIIb) as well as the catalyst have been discussed to a great extend already above.

This reaction mixture results in the formation of the compound of the formula (I'). The compound of the formula (I') can be isomerized in the presence of a base or an acid, preferable a base, to a diene ketone of the formula (IV)

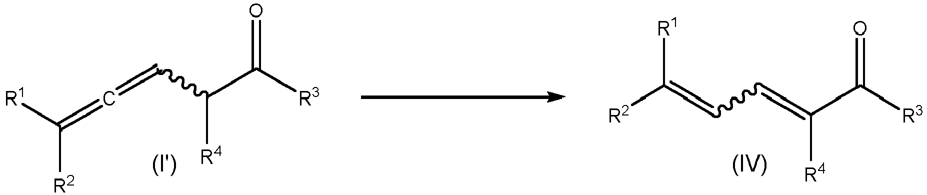

Thus, the present invention relates in a further aspect to a process for the manufacture of a diene ketone of the formula (IV) 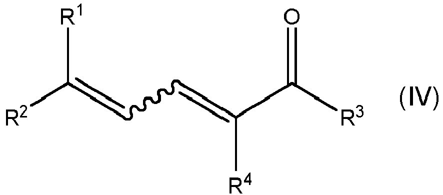 comprising the steps
a) preparing a compound of the formula (I') as discussed above already in great detail;
   followed by
b) isomerization of compound of the formula (I') in the presence of a base or an acid, preferable a base, to yield the diene ketone of the formula (IV).

The isomerization of the compound of the formula (I') to the compound of the formula (IV) are principally known to the person skilled in the art, e.g. from US 3,330,867 and WO 2017/131607, the entire content both of which is hereby incorporated by reference.

The base used in the isomerization step, i.e. step b) is preferably hydroxide or carbonate or hydrogen carbonate, preferably a hydroxide, of an alkali or earth alkali metal, particularly KOH or NaOH.

In another preferred embodiment, the base is a basic ion exchange resin, preferably the basic anion exchange resins Amberlite^{®} IRA 900, Dowex^{®} MSA-1, Diaion ^{®} HPA25 or PA308 as well as Amberlysts^{®} A260H, XE-4, XE-8, XE-8 new and XE-10 from DOW and equivalent resins with same chemical structure and similar physico-chemical properties.

The temperature in this isomerization step is preferably below 30°C, particularly between -10°C and 25°C, preferably in the range of 0°C to 10°C.

Said isomerization step is preferably performed in a C₁₋₆-alcohol, particularly in methanol, or in aqueous media.

In a further aspect, the present invention relates also to reaction mixture comprising
α) a compound of the formula (IIb), 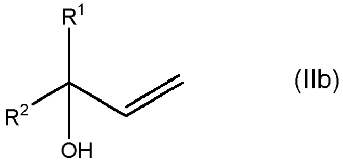
β) a compound of the formula (IIIa) or (IIIb) 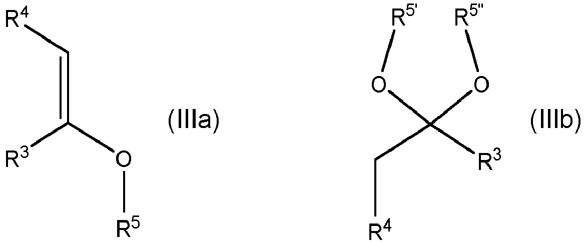
γ) an adduct of a tertiary amine with SO₃ as catalyst,
wherein the tertiary amine of the adduct is
either
a pyridinic compound of the formula (V) 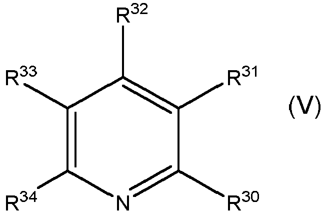 wherein R³⁰, R³¹, R³², R³³ and R³⁴ represent independently from each other either H or a linear or branched C₁₋₁₂-alkyl or C₅₋₁₂-cycloalkyl group;
   or
a tertiary amine of the formula (X) 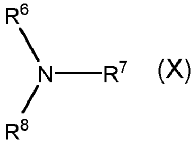 wherein R⁶ and R⁷ and R⁸ represent independently from each a linear or branched C₁₋₁₀-alkyl group
   wherein
   R¹ represents a methyl or ethyl group;
   R² represents a saturated or unsaturated linear or branched or cyclic hydrocarbyl group with 1 to 46 C atoms;
   R³ represents a methyl or an ethyl group;
   R⁴ represents H or methyl or an ethyl group;
   R⁵ represents a linear or branched C₁₋₁₀-alkyl group, particularly a methyl or an ethyl group;
   R^{5'} and R^{5"} represent
      either a linear or branched C₁₋₁₀-alkyl group, particularly a methyl or an ethyl group;
      or R^{5'} and R^{5"} form together a linear or branched C₁₋₁₀-alkylene group, particularly an ethylene or propylene group.

The compound of the formula (IIb) and of the formula (IIIa) or (IIIb) as well as the catalyst have been discussed to a great extend already above.

This reaction mixture results in the formation of the compound of the formula (I").

Said above processes allows the manufacturing of the compound of the formula (I) or (IV), respectively, in high yields and selectivities in respect to the compound of the formula (IIa) or (IIb). Hence, the present invention is offering big advantages over the processes known to person skilled in the art.

### Examples

The present invention is further illustrated by the following experiments.

### Experimental series 1

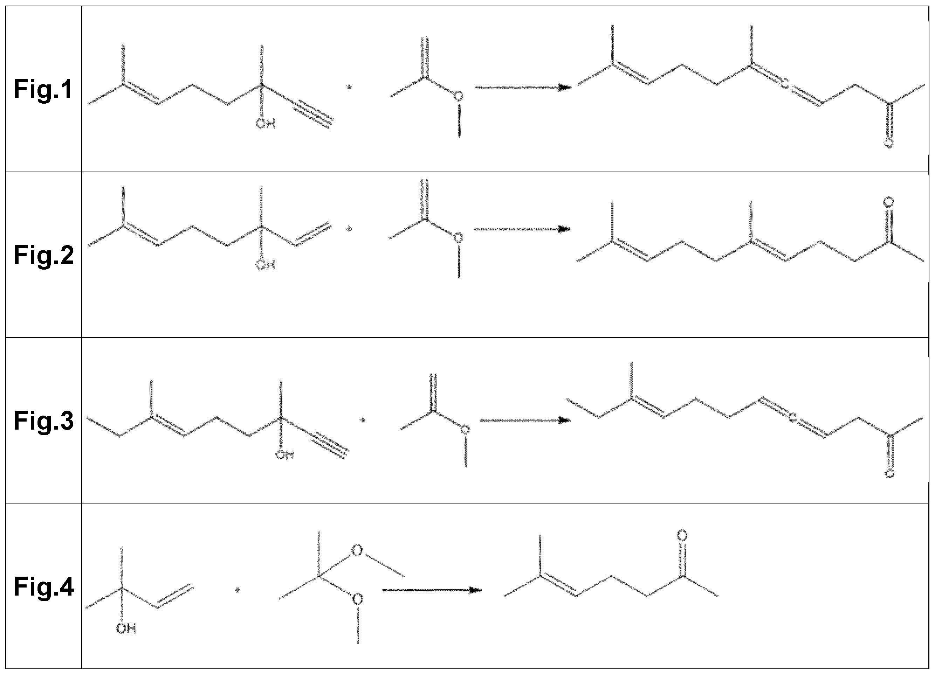

3,7-dimethyloct-6-en-1-yn-3-ol ("*DLL*") was mixed with 4 equivalents of isopropenyl methyl ether ("*IPM*") in the presence of the respective catalyst in the amount as given in table 1, and stirred at a temperature of 115 °C during 100 minutes. The respective product, i.e. 6,10-dimethylundeca-4,5,9-trien-2-one, was obtained in the yield and selectivity as indicated in table 1 (see figure 1).

**Table 1. Formation of 6,10-dimethylundeca-4,5,9-trien-2-one from 3,7-dimethyloct-6-en-1-yn-3-ol (DLL) and isopropenyl methyl ether (IPM) using different catalysts at a pressure of 8.5 bara.**

| Example | Catalyst | Amount¹ [mol%] | Yield [%] | Selectivity [%] |
|---|---|---|---|---|
| ***1*** | pyridine·SO₃ | 0.1 | 97 | 97 |
| ***2*** | triethylamine·SO₃ | 0.5 | 62 | 98 |

| | | | | |
|---|---|---|---|---|
| ¹amount of catalyst relative to *DLL* | | | | |

The 6,10-dimethylundeca-4,5,9-trien-2-one from table 1 was quantitatively isomerized to 6,10-dimethylundeca-3,5,9-trien-2-one, by the reaction as indicated in example 2 in WO 2011/131607.

### Experimental series 2

3,7-dimethylocta-1,6-dien-3-ol ("LL") was mixed with the number of equivalents given in table 2 of isopropenyl methyl ether ("*IPM*") in the presence of 0.01 mol-% (relative to the amount of 3,7-dimethylocta-1,6-dien-3-ol ("*LL*")) of the respective catalyst, and stirred at a temperature of 150°C during 16 hours. The respective product, i.e. 6,10-dimethylundeca-5,9-dien-2-one, was obtained in the yield and selectivity as indicated in table 2 (see figure 2).

**Table 2. Formation of 6,10-dimethylundeca-5,9-dien-2-one from 3,7-dimethylocta-1,6-dien-3-ol ("LL") and isopropenyl methyl ether (IPM) using different catalysts at a pressure of 8.5 bara.**

| Example | Catalyst | Amount IPM¹ [eq] | Yield [%] | Selectivity [%] |
|---|---|---|---|---|
| ***3*** | triethylamine·SO₃ | 2.1 | 41 | 44 |
| ***4*** | triethylamine·SO₃ | 2.5 | 44 | 44 |
| ***5*** | pyridine·SO₃ | 2.1 | 38 | 28 |
| ***6*** | pyridine·SO₃ | 2.5 | 43 | 44 |

| | | | | |
|---|---|---|---|---|
| ¹amount of *IPM* relative to *LL* | | | | |

The 6,10-dimethylundeca-5,9-dien-2-one from table 2 was quantitatively isomerized to 6,10-dimethylundeca-4,9-dien-2-one, by the reaction as indicated in example 2 in WO 2011/131607.

### Experimental series 3

3,7-dimethylnon-6-en-1-yn-3-ol ("EDLL") was mixed with 4 equivalents of isopropenyl methyl ether (*"IPM"*) in the presence of 0.1 mol-% the adduct of pyridine and SO₃ (relative to the amount of *EDLL*) and stirred at a temperature of 115 °C during 100 minutes. The respective product, i.e. 6,10-dimethyldodeca-4,5,9-trien-2-one, was obtained in the yield and selectivity as indicated in table 3 (see figure 3).

**Table 3. Formation of 6,10-dimethyldodeca-4,5,9-trien-2-one from 3,7-dimethylnon-6-en-1-yn-3-ol ("EDLL") and isopropenyl methyl ether (IPM) using pyridine/SO₃ adduct at a pressure of 8.5 bara.**

| Example | Catalyst | Amount¹ [mol%] | Yield [%] | Selectivity [%] |
|---|---|---|---|---|
| ***7*** | pyridine·SO₃ | 0.1 | 97 | 98 |

| | | | | |
|---|---|---|---|---|
| ¹ amount of catalyst relative to *EDLL* | | | | |

The 6,10-dimethyldodeca-4,5,9-trien-2-one from table 3 was quantitatively isomerized to 6,10-dimethyldodeca-3,5,9-trien-2-one by the reaction as indicated in example 2 in WO 2011/131607.

### Experimental series 4

2-methylbut-3-en-2-ol (*"MBE"*) was mixed with 8.0 equivalents of 2,2-dimethoxypropane ("*DMP*") in the presence of 0.005 mol-% (relative to the amount of *MBE*) of pyridine·SO₃ as catalyst, and stirred at a temperature of 150°C during 8 hours. The respective product, i.e. 6-methylhept-5-en-2-one was obtained in the yield and selectivity as indicated in table 4 (see figure 4).

**Table 4. Formation of 6-methylhept-5-en-2-one from MBE and DMP.**

| Example | Catalyst | Amount DMP¹ [eq] | Conversion [%] | Yield [%] | Selectivity [%] |
|---|---|---|---|---|---|
| ***8*** | pyridine·SO₃ | 8.0 | 68 | 23 | 34 |

| | | | | | |
|---|---|---|---|---|---|
| ¹amount of *DMP* relative to *MBE.* | | | | | |

### Experimental series 5

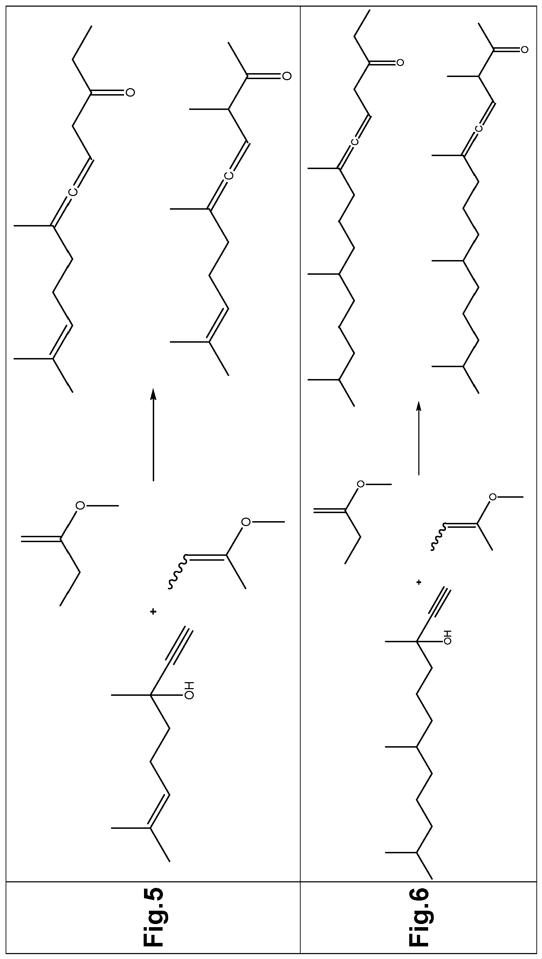

3,7-dimethyloct-6-en-1-yn-3-ol (= dehydrolinalool, *"DLL"*) was mixed with 2.6 equivalents of butenyl methyl ether (mixture of 2-methoxybut-1-ene / (E)-2-methoxybut-2-ene / (Z)-2-methoxybut-2-ene = 53/37/10 in the presence of the respective catalyst in the amount as given in table 5 and stirred at a temperature of 80-95 °C during the reaction time as indicated in table 5. The respective products, i.e. 7,11-dimethyldodeca-5,6,10-trien-3-one (=DMDTO) and 3,6,10-trimethylunde-ca-4,5,9-trien-2-one (=TMUTO), were obtained in the conversion, yield and selectivity as indicated in table 5 (see figure 5).

**Table 5. Formation of 3,6,10-trimethylundeca-4,5,9-trien-2-one (=TMUTO) and 7,11-dimethyldodeca-5,6,10-trien-3-one (DMDTO) from 3,7-dimethyl-oct-6-en-1-yn-3-ol and butenyl methyl ether using tertiary amine / SO₃ adduct catalysts and pressure of 1 bara.**

| Example | Catalyst¹ | Amount¹ [mol%] | Reaction time [h] | Conversion [%] | TMUTO/DMDTO | Yield [%] | Selectivity [%] |
|---|---|---|---|---|---|---|---|
| ***9*** | triethylamine·SO₃ | 0.5 | 20 | 59 | 33/67 | 46 | 77 |
| ***10*** | pyridine·SO₃ | 4 | 2.5 | 99 | 97/3 | 68 | 69 |
| ***11*** | pyridine·SO₃ | 0.5 | 20 | 99 | 87/13 | 52 | 53 |
| ***Ref.1*** | (Et₃NH)₂HPO₄ | 4 | 2.5 | 0 | n.a.2 | 0 | n.a.2 |
| ***Ref.2*** | (Et₃NH)₂HPO₄ | 0.5 | 20 | 4.4 | 56/44 | 1 | 20 |

| | | | | | | | |
|---|---|---|---|---|---|---|---|
| ¹ amount of catalyst relative to *DLL* ² n.a.= not applicable | | | | | | | |

The 3,6,10-trimethylundeca-4,5,9-trien-2-one and 7,11-dimethyldodeca-5,6,10-trien-3-one from table 5 were quantitatively isomerized to 3,6,10-trimethylundeca-3,5,9-trien-2-one, respectively 7,11-dimethyldodeca-4,6,10-trien-3-one, by the reaction as indicated in example 2 in WO 2011/131607.

### Experimental series 6

3,7,11-trimethyldodec-1-yn-3-ol was mixed with 2.6 equivalents of butenyl methyl ether (mixture of 2-methoxybut-1-ene / (E)-2-methoxybut-2-ene / (Z)-2-methoxybut-2-ene = 53/37/10) in the presence of the respective ammonium catalyst in the amount as given in table 6 and stirred at a temperature of 80-95 °C during the reaction time as indicated in table 6. The respective products, i.e.

7,11,15-trimethylhexadeca-5,6-dien-3-one (TMHDO) and 3,6,10,14-tetramethyl-pentadeca-4,5-dien-2-one (TMPDO), were obtained in the yield and selectivity as indicated in table 6 (see figure 6).

**Table 6. Formation of 7,11,15-trimethylhexadeca-5,6-dien-3-one (TMHDO) and 3,6,10,14-tetramethylpentadeca-4,5-dien-2-one (TMPDO) from 3,7,11-trimethyldodec-1-yn-3-ol and butenyl methyl ether using different catalysts and pressure of 1 bara.**

| Example | Catalyst | Amount¹ [mol%] | Reaction time [h] | Conversion [%] | TMPDO/TMHDO | Yield [%] | Selectivity [%] |
|---|---|---|---|---|---|---|---|
| ***12*** | triethylamine·SO₃ | 0.5 | 2.5 | 68 | 31/69 | 57 | 84 |
| ***13*** | pyridine·SO₃ | 4 | 20 | 99 | 92/8 | 78 | 79 |
| ***14*** | pyridine·SO₃ | 0.5 | 20 | 100 | 97/17 | 71 | 71 |
| ***Ref.3*** | (Et₃NH)₂HPO₄ | 4 | 2.5 | 0.1 | n.a.2 | 0 | n.a.2 |
| ***Ref.4*** | (Et₃NH)₂HPO₄ | 0.5 | 20 | 68.8 | 32/68 | 55.6 | 81 |

| | | | | | | | |
|---|---|---|---|---|---|---|---|
| ¹ amount of catalyst relative to 3,7,11-trimethyldodec-1-yn-3-ol ² n.a.= not applicable | | | | | | | |

The 7,11,15-trimethylhexadeca-5,6-dien-3-one and 3,6,10,14-tetramethyl-pentadeca-4,5-dien-2-one from table 6 were quantitatively isomerized to 7,11,15-trimethylhexadeca-4,6-dien-3-one, respectively 3,6,10,14-tetramethylpentadeca-3,5-dien-2-one, by the reaction as indicated in example 2 in WO 2011/131607.

## Claims

1. A process for the manufacture of an unsaturated ketone of the formula (I) 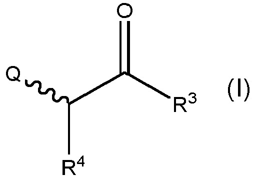 by reaction of
a compound of the formula (IIa) or (IIb) with a compound of the formula (IIIa) or (IIIb) 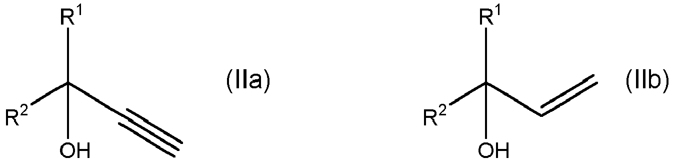 with a compound of the formula (IIIa) or (IIIb) 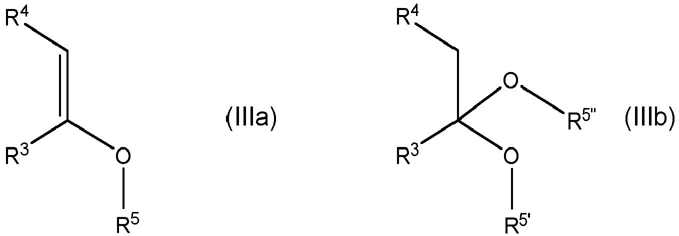 in the presence of an adduct of a tertiary amine with SO₃ as catalyst; wherein
Q represents a group of the formula 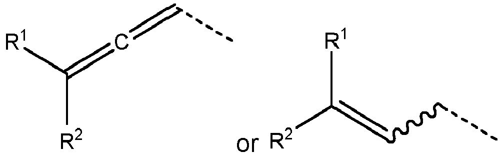
R¹ represents a methyl or ethyl group;
R² represents a saturated or unsaturated linear or branched or cyclic hydrocarbyl group with 1 to 46 C atoms;
R³ represents a methyl or an ethyl group;
R⁴ represents H or methyl or an ethyl group;
R⁵ represents a linear or branched C₁₋₁₀-alkyl group, particularly a methyl or an ethyl group;
R^{5'} and R^{5"} represent
either a linear or branched C₁₋₁₀-alkyl group, particularly a methyl or an ethyl group;
or R^{5'} and R^{5"} form together a linear or branched C₁₋₁₀-alkylene group, particularly an ethylene or propylene group;
and
wherein the tertiary amine of said adduct is
either
pyridine
or
a tertiary amine of the formula (X) 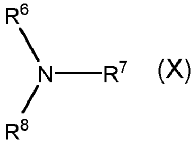 wherein R⁶ and R⁷ and R⁸ represent independently from each a linear or branched C₁₋₁₀-alkyl group
and
wherein the wavy line represents a carbon-carbon bond and which when linked to the carbon-carbon double bond is either in the Z or in the E-configuration;
and any dotted line represents the bond by which the substituent Q is bound to the rest of the compound of the formula (I).

2. The process according to any one of the proceeding claims, **characterized in that** R¹ represents a methyl group.

3. The process according to any one of the preceding claims 1-2, **characterized in** R² = methyl.

4. The process according to any one of the preceding claims 1-2, **characterized in that** R² is selected from the group consisting of the formula (R2-I), (R2-II), (R2-III) and (R2-IV), 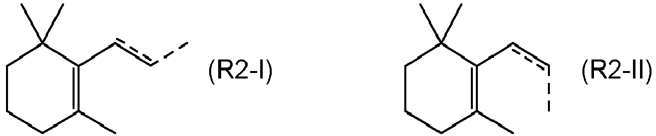 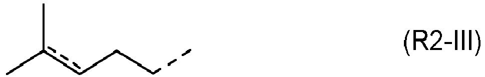 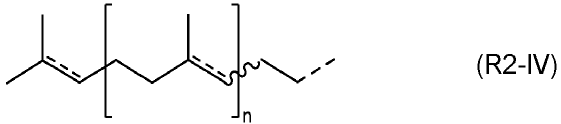 wherein the dotted line represents the bond by which the substituent of the formula (R2-I), (R2-II), (R2-III) or (R2-IV) is bound to the rest of the compound of the formula (I) or formula (IIa) or (IIb);
and wherein any double bond having dotted line (------) represents independently from each other either a single carbon-carbon bond or a double carbon-carbon bond;
and wherein any wavy line represents independently from each other a carbon-carbon bond which when linked to the carbon-carbon double bond is either in the Z or in the E-configuration;
and wherein n represents 1, 2, 3 or 4, particularly 1 or 2.

5. The process according to any one or more of the preceding claims, **characterized in that** the molar ratio of the compound of the formula (IIa) or (IIb) to the compound of the formula (IIIa) or (IIIb) is ranging from 1:15 to 1:1, and in case of using the compound of the formula (IIIa)
said ratio is more preferred in the range from 1:5 to 1:2, more preferably ranging from 1:3.5 to 1:2, most preferably ranging from 1:3 to 1:2, particularly ranging from 1:2.5 to 1:2;
or in case of using the compound of the formula (IIIa)
said ratio is more preferred in the range from 1:10 to 1:2, more preferably ranging from 1:8 to 1:3, most preferably ranging from 1:8 to 1:5.

6. The process according to any one of the preceding claims, **characterized in that** the amount of the adduct of a tertiary amine with SO₃ is ranging from 0.01 - 1 mol-%, preferably ranging from 0.02 - 0.6 mol-%, more preferably ranging from 0.05 - 0.6 mol-%, based on the amount of the compound of the formula (IIa) or (IIb).

7. The process according to any one of the preceding claims 1-6, **characterized in that** the adduct of a tertiary amine with SO₃ is

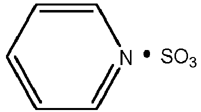

8. The process according to any one of the preceding claims 1-6, **characterized in that** the adduct of a tertiary amine with SO₃ is

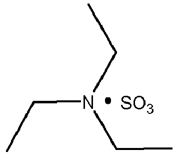

9. The process according to any one of the preceding claims, **characterized in that** the compound of the formula (I) is formula (I") 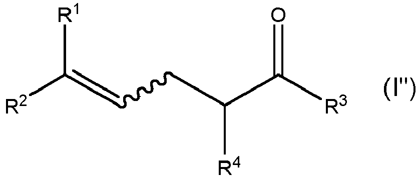 and is prepared by reaction of the compound of the formula (IIb) with a compound of the formula (IIIa) or (IIIb), preferably with the compound of the formula (IIIa), 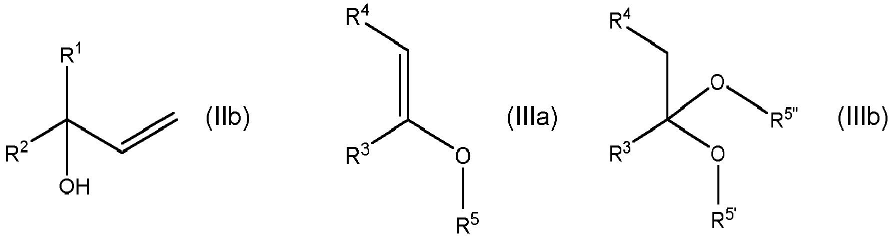 in the presence of the adduct of a tertiary amine with SO₃ as catalyst.

10. The process according to any one of the preceding claims, **characterized in that** the compound of the general formula (I) is selected from the group consisting of 6-methyl-5-hepten-2-one, 6-methyl-5-octen-2-one, 6,10-dimethyl-5,9-undecadien-2-one, 6,10-dimethyl-5-undecen-2-one, 6,10,14-trimethylpentadeca-5,9-dien-2-one, 6,10,14-trimethylpentadeca-5,13-dien-2-one and 6,10,14-trimethyl-5,9,13-pentadeca-trien-2-one, preferably 6-methyl-5-hepten-2-one.

11. The process according to any one of the preceding claims 1-8, **characterized in that** the compound of the formula (I) is formula (I') 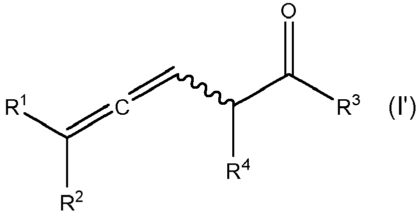 and is prepared by reaction of the compound of the formula (IIa) with a compound of the formula (IIIa) or (IIIb), preferably with the compound of the formula (IIIa), 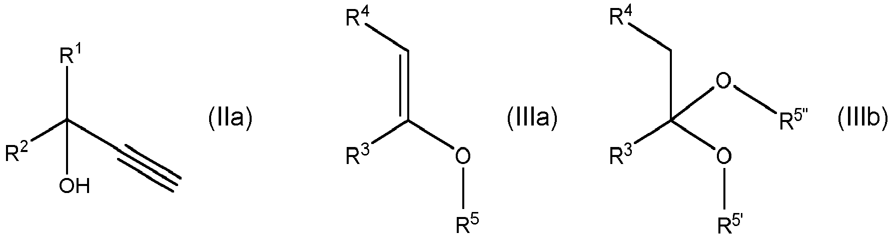 in the presence of the adduct of a tertiary amine with SO₃ as catalyst.

12. The process according to any one of the preceding claims 1-8 or 11, **characterized in that** the compound of the general formula (I) is selected from the group consisting of 6-methylhepta-4,5-dien-2-one, 6,10-dimethylundeca-4,5-dien-2-one, 6,10-dimethylundeca-4,5,9-trien-2-one, 6,10,14-trimethylpentadeca-4,5,9,13-tetraen-2-one, 6,10,14-trimethylpentadeca-4,5,9-trien-2-one, 6,10,14-trimethylpentadeca-4,5,13-trien-2-one and 6,10,14-trimethylpentadeca-4,5-dien-2-one.

13. A reaction mixture comprising
i) a compound of the formula (IIa), 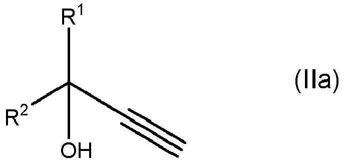
ii) a compound of the formula (IIIa) or (IIIb) 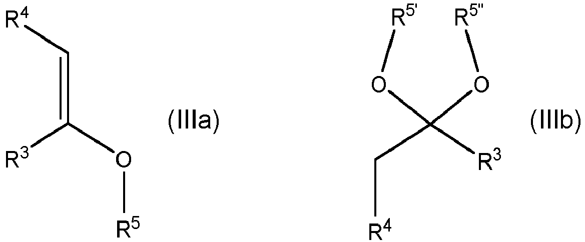 and
iii) an adduct of a tertiary amine with SO₃ as catalyst, wherein the tertiary amine of the adduct is either
a pyridinic compound of the formula (V) 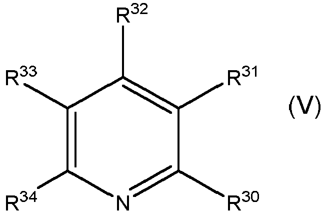 wherein R³⁰, R³¹, R³², R³³ and R³⁴ represent independently from each other either H or a linear or branched C₁₋₁₂-alkyl or C₅₋₁₂-cycloalkyl group;
or
a tertiary amine of the formula (X) 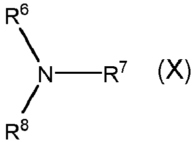 wherein R⁶ and R⁷ and R⁸ represent independently from each a linear or branched C₁₋₁₀-alkyl group
wherein
R¹ represents a methyl or ethyl group;
R² represents a saturated or unsaturated linear or branched or cyclic hydrocarbyl group with 1 to 46 C atoms;
R³ represents a methyl or an ethyl group;
R⁴ represents H or methyl or an ethyl group;
R⁵ represents a linear or branched C₁₋₁₀-alkyl group, particularly a methyl or an ethyl group;
R^{5'} and R^{5"} represent
either a linear or branched C₁₋₁₀-alkyl group, particularly a methyl or an ethyl group;
or R^{5'} and R^{5"} form together a linear or branched C₁₋₁₀-alkylene group, particularly an ethylene or propylene group.

14. A reaction mixture comprising
α) a compound of the formula (IIb), 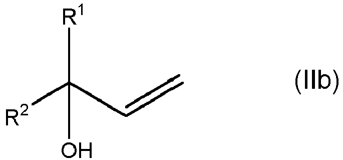
β) a compound of the formula (IIIa) or (IIIb) 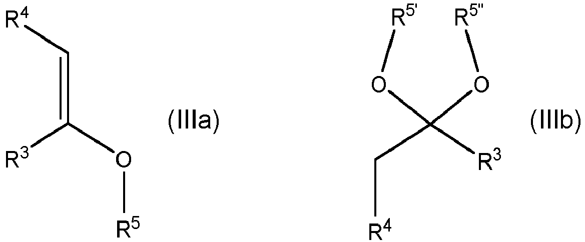
γ) an adduct of a tertiary amine with SO₃ as catalyst, wherein the tertiary amine of the adduct is either
a pyridinic compound of the formula (V) 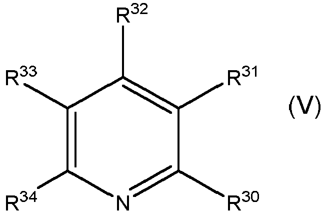 wherein R³⁰, R³¹, R³², R³³ and R³⁴ represent independently from each other either H or a linear or branched C₁₋₁₂-alkyl or C₅₋₁₂-cycloalkyl group;
or
a tertiary amine of the formula (X) 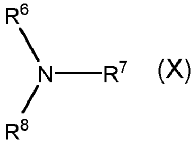 wherein R⁶ and R⁷ and R⁸ represent independently from each a linear or branched C₁₋₁₀-alkyl group
wherein
R¹ represents a methyl or ethyl group;
R² represents a saturated or unsaturated linear or branched or cyclic hydrocarbyl group with 1 to 46 C atoms;
R³ represents a methyl or an ethyl group;
R⁴ represents H or methyl or an ethyl group;
R⁵ represents a linear or branched C₁₋₁₀-alkyl group, particularly a methyl or an ethyl group;
R^{5'} and R^{5"} represent
either a linear or branched C₁₋₁₀-alkyl group, particularly a methyl or an ethyl group;
or R^{5'} and R^{5"} form together a linear or branched C₁₋₁₀-alkylene group, particularly an ethylene or propylene group.

15. A process for the manufacture of an diene ketone of the formula (IV) 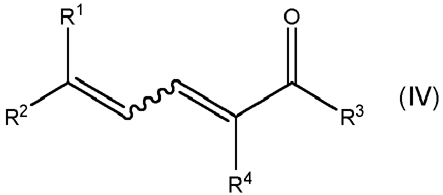 comprising the steps
a) preparing a compound of the formula (I') according to anyone of the preceding claims 11 or 12;
followed by
b) isomerization of compound of the formula (I') in the presence of a base or an acid, preferable a base, to yield the diene ketone of the formula (IV).
